# EUROPEAN PATENT SPECIFICATION

(11) **EP 4 394 016 B1**
(45) Date of publication and mention of the grant of the patent: **20.08.2025**
(21) Application number: 23220786.0
(22) Date of filing: 29.12.2023
(51) Int. Cl.: C09K 11/07, G01N 33/533

(54) **CHEMILUMINESCENT SUBSTRATE SOLUTION, CHEMILUMINESCENCE ENHANCER AND USE THEREOF**
CHEMILUMINESZENTE SUBSTRATLÖSUNG, CHEMILUMINESZENZVERSTÄRKER UND VERWENDUNG DAVON
SOLUTION DE SUBSTRAT CHIMILUMINESCENT, ACTIVATEUR DE CHIMIOLUMINESCENCE ET UTILISATION ASSOCIÉE

(30) Priority: 30.12.2022 CN 202211743585
(43) Date of publication of application: 03.07.2024
(73) Proprietor: Shenzhen Mindray Bio-Medical Electronics Co., Ltd., Shenzhen, Guangdong 518057 (CN)
(72) Inventor: WANG, Junlin, Shenzhen, 518057 (CN); ZHAN, Chengxiong, Shenzhen, 518057 (CN); LI, Ke, Shenzhen, 518057 (CN)
(74) Representative: KIPA AB

(56) References cited:
- US-A1- 2007 059 786

## Description

### TECHNICAL FIELD

The disclosure relates to the field of in-vitro diagnostic immunoassay, and in particular to an alkaline phosphatase-catalyzed chemiluminescence enhancer, a chemiluminescent substrate solution and the use thereof.

### BACKGROUND

Chemiluminescence refers to a phenomenon that in the process of a chemical reaction, a specific substance absorbs part of chemical energy to reach an excited state, and then returns to a ground state, during which the energy is released in the form of photons, resulting in luminescence. Chemiluminescence immunoassay (CLIA) refers to an analytical technique that combines a high-sensitivity chemiluminescence technique with a high-specificity immune response for the detection of antigens, antibodies, hormones, fatty acids, vitamins and drugs. Chemiluminescence immunoassay has the high sensitivity of chemiluminescence and has the characteristics of simple operation and rapid response of enzyme-linked immunoassay, which is readily to standardize experimental operations, and now has been widely used in biological and medical studies and clinical diagnosis.

The substrate luminescence system is the most important part in chemiluminescence immunoassay. Currently, horseradish peroxidase and alkaline phosphatase catalytic systems are the main ones used in chemiluminescence immunoassay. The alkaline phosphatase catalytic system is more widely used.

However, in the alkaline phosphatase-catalyzed chemiluminescence system, an enhancer needs to be added to improve the luminescence efficiency. Enhancers are generally imported materials, which are expensive, and have disadvantages such as a long time to reach plateau, a slow response speed, a high level of background, a low sensitivity, and not capable of meeting the detection requirements for some high-sensitivity projects.

Therefore, a chemiluminescent substrate solution suitable for an alkaline phosphatase catalyzed system still needs further improvements.

US2007059786 discloses a chemiluminescence enhancer, comprising similar fluorescent agents.

### SUMMARY

An object of the disclosure is to provide a fluorescent agent suitable for an alkaline phosphatase-catalyzed chemiluminescence system, so as to obtain a chemiluminescence enhancer and a chemiluminescent substrate solution that have a simple formulation, a rapid reaction rate, a short time to reach plateau and a high sensitivity.

For this purpose, the first aspect of the disclosure provides a chemiluminescent substrate solution, which comprises a chemiluminescent substrate and a chemiluminescence enhancer. The chemiluminescence enhancer comprises a fluorescent agent and a surfactant, in which the fluorescent agent comprises one or more carboxyfluoresceins selected from compounds represented by general formula I:

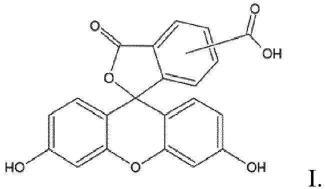

In an embodiment, the fluorescent agent is selected from 5-carboxyfluorescein, 6-carboxyfluorescein or 5(6)-carboxyfluorescein; in which 5-carboxyfluorescein is 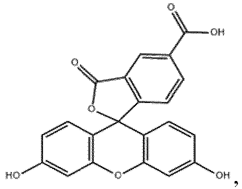
6-carboxyfluorescein is 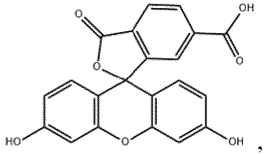 and
5(6)-carboxyfluorescein is a mixture of 5-carboxyfluorescein and 6-carboxyfluorescein.

In an embodiment, the chemiluminescent substrate is one selected from dioxetane-based compounds and acridine-based compounds.

In an embodiment, the chemiluminescent substrate is one selected from dioxetane-based compounds each having a spiro-adamantane substituent, and preferably selected from AMPPD, CSPD, ADP-STAR, CDP-STAR and TFE-AMPPD; or the chemiluminescent substrate is one selected from chlorinated dioxetane-based compounds each having a spiro-adamantane substituent, and preferably selected from CSPD, ADP-STAR and CDP-STAR.

In an embodiment, the surfactant is one or more selected from a cationic surfactant, an anionic surfactant, a zwitterionic surfactant and a non-ionic surfactant. Preferably selected froma cationic surfactant of a water-soluble polymer quaternary ammonium salt, a quaternary sulfonium salt and a quaternary phosphonium salt, more preferably selected from the cationic surfactant of a water-soluble polymer quaternary ammonium salt. More preferably is polyvinylbenzyltrimethylammonium chloride.

In an embodiment, the chemiluminescent substrate is one of CSPD, ADP-STAR and CDP-STAR, and the surfactant is polyvinylbenzyltrimethylammonium chloride. In a particular embodiment, CSPD or ADP-STAR or CDP-STAR has a concentration of 50-500 mg/L, and polyvinylbenzyltrimethylammonium chloride has a concentration of 1-10 g/L.

In an embodiment, in the chemiluminescent substrate solution, the chemiluminescent substrate has a concentration of 50-500 mg/L, and preferably 150-250 mg/L; the fluorescent agent has a concentration of 30-500 mg/L, and preferably 150-250 mg/L; and the surfactant has a concentration of 1-10 g/L, and preferably 3-7 g/L.

In an embodiment, the chemiluminescent substrate solution further includes a buffer and a preservative.

In an embodiment, the buffer has a pH of 7.1-10.6, and preferably 9-10.

In an embodiment, the buffer is at least one selected from Tris-HCl, AMP-HCl, AMPD-HCl, DEA-HCl, CHES-HCl, boric acid-NaOH and glycine-NaOH.

In an embodiment, the preservative is at least one selected from sodium azide, Proclin series, potassium sorbate and sodium benzoate.

In an embodiment, the chemiluminescent substrate solution is used in an alkaline phosphatase-catalyzed chemiluminescence system.

In an embodiment, the chemiluminescent substrate solution is used in a chemiluminescence system with an alkaline phosphatase having a concentration of less than 10^-19 mol/L.

The second aspect of the disclosure provides a chemiluminescence enhancer, which comprises a fluorescent agent and a surfactant. The fluorescent agent comprises one or more of carboxyfluoresceins selected from compounds represented by general formula I:

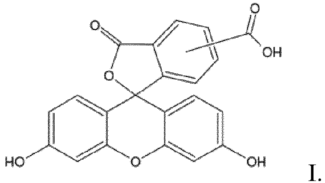

In an embodiment, the fluorescent agent is selected from 5-carboxyfluorescein, 6-carboxyfluorescein or 5(6)-carboxyfluorescein; in which
5-carboxyfluorescein is 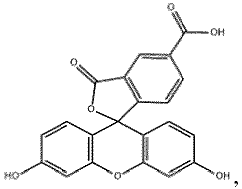
6-carboxyfluorescein is 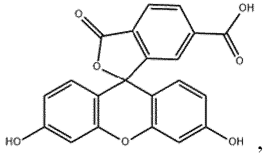 and
5(6)-carboxyfluorescein is a mixture of 5-carboxyfluorescein and 6-carboxyfluorescein.

In some embodiment, the surfactant is one or more selected from a cationic surfactant, an anionic surfactant, a zwitterionic surfactant and a non-ionic surfactant. Preferably the surfactant is one or more selected from a cationic surfactant of a water-soluble polymer quaternary ammonium salt, a quaternary sulfonium salt or a quaternary phosphonium salt, and more preferably selected from the cationic surfactant of the water-soluble polymer quaternary ammonium salt. More preferably the surfactant is polyvinylbenzyltrimethylammonium chloride

The third aspect of the disclosure provides use of a carboxyfluorescein in enhancing chemiluminescence of a dioxetane derivative catalyzed by alkaline phosphatase, in which the carboxyfluorescein is selected from compounds represented by general formula I:

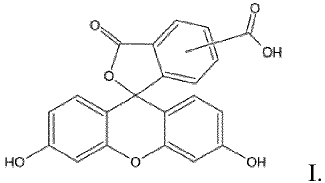

The disclosure achieves a faster luminescence efficiency of a chemiluminescent substrate solution, and achieves a shorter time to reach a luminescence plateau, a longer duration of the luminescence plateau, high detection sensitivity and a good stability, thereby improving the detection speed and the detection accuracy, and shortening the detection time. The raw materials used in the disclosure are readily available, have stable performance, good antimicrobial performance, low cost, are suitable for industrial production, and are capable of replacing original imported reagents, thereby reducing the cost of clinical testing.

### BRIEF DESCRIPTION OF THE DRAWINGS

FIG. 1 shows photodynamic curves of chemiluminescent substrate solutions 1-1 to 1-14 combined with different fluorescent agent of example 1 of the disclosure;
FIG. 2 is a signal-to-noise ratio histogram of chemiluminescent substrate solutions 2-1 to 2-9 combined with different surfactants of example 2 of the disclosure; and
FIG. 3 shows photodynamic curves of chemiluminescent substrate solutions 3-1, 3-2, 3-3 and 3-4 in which a carboxyfluorescein is combined with different chemiluminescent substrates of example 3 of the disclosure.

### DESCRIPTION OF THE PREFERRED EMBODIMENTS

The technical solutions of the disclosure will be clearly and completely described below in conjunction with specific embodiments and drawings of the disclosure. It is apparent that the embodiments described are merely some of the embodiments of the disclosure rather than all the embodiments. Any other embodiments obtained by those of ordinary skill in the art based on the embodiments of the disclosure without any inventive effort shall fall within the protection scope of the disclosure.

Throughout the specification, unless otherwise specified, the terms used herein should be understood as the meanings commonly used in the art. Therefore, unless otherwise defined, all the technical and scientific terms used herein have the same meaning as commonly understood by those of skill in the art to which the disclosure belongs. In the event of a conflict, this specification takes precedence.

In this application, the terms "comprise", "include" or any other variation thereof are intended to cover non-exclusive inclusion, so that a method or product comprising a series of elements comprises not only explicitly recorded elements, but also other elements not explicitly listed, or elements inherent in implementing the method or product.

Unless otherwise specified, the singular forms "a/an" and "the/said" as used herein include the plural of the noun referred to.

The "chemiluminescent substrate" mentioned herein, unless otherwise indicated, refers to a compound that participates in energy transfer in a chemiluminescence reaction and ultimately releases energy in the form of emitted photons, and is also known as a chemiluminescent reagent or a luminescent substrate. Common chemiluminescent substrates mainly include aminobenzoyldrazide compounds (e.g., luminol and isoluminol derivatives), acridine compounds (e.g., acridinium esters), electochemiluminescence system compounds (e.g., tris-2-2'-bipyridyl ruthenium and tripropylamine) and dioxetane compounds. Isoluminol and acridinium esters are used as tracer-molecule for direct labeling, and belong to flash-type chemiluminescence reactions. Luminol and dioxetane compounds rely on enzymes which serve as tracer-molecule markers, and belong to enzymatic glow-type chemiluminescence reactions. Dioxetane compounds are the latest ultra-sensitive substrates for an alkaline phosphatase (AP). With the catalytic hydrolysis effect of the alkaline phosphatase (AP) in a suitable buffer solution, the signal generated by the decomposition of a dioxetane compound can last for 20 hours or more, making the compound an ideal chemiluminescent substance.

The "chemiluminescence enhancer" mentioned herein, unless otherwise indicated, refers to a substance added to a conventional chemiluminescence system for enhancing the luminescence signal or extend the luminescence time of the system. In general, chemiluminescence enhancers can significantly improve the analytical sensitivity, stability of results, and convenience of use of chemiluminescence immunoassay by promoting electron activation.

It is well-known that a quenching reaction tends to occur in a liquid medium, particularly in an aqueous medium. In chemiluminescence immunoassay, many of the test samples are usually biological samples, and measurements of the samples by this method are usually performed in an aqueous medium. However, the quenching reaction in an aqueous medium sometimes significantly reduces chemiluminescence produced by a substrate. Moreover, some test samples (such as nucleic acids, virus antibodies, and proteins) need to be detected at a low level, and the decrease of chemiluminescence caused by the quenching reaction and inevitable background signals in a system make the sensitivity of the detection greatly reduced, thus making it impossible to achieve the detection of a sample with a very low level.

On this basis, studies have shown that in order to reduce quenching reactions and improve luminescence signals, a chemiluminescence system can commonly be added with some naturally occurring or synthetic water-soluble polymers, water-soluble enhancers (see U.S. Patent No. US 4978614 A), or surfactants (see U.S. Patent No. US 7276343 B2).

However, the effect of reducing quenching reactions and improving luminescence signals by simply adding some naturally occurring or synthetic water-soluble polymers, water-soluble enhancers, or surfactants to a luminescence system as described above is still relatively limited, and the addition of an excess of surfactants tends to produce excessive foams, which affects the accuracy of the detection for a luminescence value.

In another aspect, studies show that chemiluminescence efficiency can also be improved by adding a "fluorescent agent" in conjunction with a "surfactant" to a luminescent system, in which the surfactant forms micelles in the solution, which results in the reduction of quenching reactions of a chemiluminescent substrate. The "fluorescent agent" mentioned herein, serving as a photon acceptor, receives the photon energy in the system through the energy transfer effect and is excited to generate light signals, thus greatly enhancing the luminescence efficiency. In the prior art, a surfactant is generally used in conjunction with a fluorescein as a chemiluminescence enhancer; however, the enhancement efficiency thereof is limited, which cannot fully satisfy the requirements of sensitivity and accuracy in the course of actual in-vitro detections or clinical testing.

On this basis, the disclosure provides a novel "fluorescent agent", i.e., a "carboxyfluorescein" mentioned herein, through extensive theoretical and experimental studies, and extensive screenings and studies on fluorescein and derivatives thereof. Accordingly, a chemiluminescent substrate solution is provided, which includes a chemiluminescent substrate and a chemiluminescence enhancer; the chemiluminescence enhancer includes a fluorescent agent and a surfactant, the fluorescent agent includes one or more carboxyfluoresceins selected from compounds of general formula I:

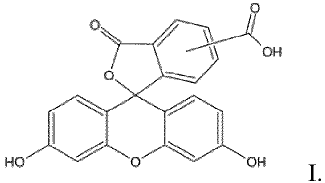

Specifically, the carboxyfluorescein of general formula I is 5-carboxyfluorescein, 6-carboxyfluorescein or 5(6)-carboxyfluorescein.
5-carboxyfluorescein is 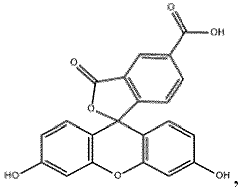
6-carboxyfluorescein is 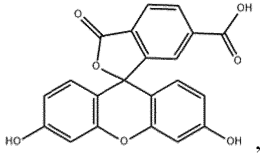 and
5(6)-carboxyfluorescein is a mixture of 5-carboxyfluorescein and 6-carboxyfluorescein in any ratio.

The following specific examples show that the use of the carboxyfluorescein of the disclosure to a chemiluminescent substrate solution can effectively improve the chemiluminescence efficiency of the chemiluminescence system, reduce the time required to reach plateau, and improve the sensitivity.

In accordance with some specific embodiments, the carboxyfluorescein in the chemiluminescent substrate solution has a concentration of 30-500 mg/L, preferably 150-250 mg/L, and particularly preferably 200 mg/L.

The surfactant in the chemiluminescence enhancer is selected from at least one of an anionic surfactant, a non-ionic surfactant, a zwitterionic surfactant and a cationic surfactant. The type of the surfactant is not specifically limited in the disclosure, and all conventionally applicable "surfactants" can be used as a component of the chemiluminescence enhancer of the disclosure. A preferred surfactant is at least one selected from water-soluble polymer quaternary ammonium salt, a quaternary sulfonium salt or a quaternary phosphonium salt (see U.S. Patent No. US 7276343 B2), more preferably the water-soluble polymer quaternary ammonium salt.

In accordance with some embodiments, the surfactant in the chemiluminescent substrate solution is one or more selected from poly(vinylbenzyltrimethylammonium chloride) (TMQ), poly(vinylbenzyltributyl ammonium chloride) (TBQ), poly(vinylbenzyldimethylbenzylammonium chloride) (BDMQ) (see US 5,112,960 and JP 8-507694 T), cetyltrimethylammonium chloride (CTAC), cetyltrimethylammonium bromide (CTAB), 3-[(3-cholamidopropyl)dimethylaminoproyl]-1-propanesulfonate (CHAPS), Triton X-405, Triton X-100 or Tween 20. A more preferred surfactant is poly(vinylbenzyltrimethylammonium chloride) (TMQ).

In accordance with some specific embodiments, the surfactant in the chemiluminescent substrate solution has a concentration of 1-10 g/L, preferably 3-7 g/L, and especially preferably 5 g/L.

In accordance with some embodiments, the chemiluminescent substrate in the chemiluminescent substrate solution is selected from dioxetane-based compounds or acridine-based compounds.

Dioxetane-based compounds or acridine-based compounds are not particularly limited in the disclosure, and all dioxetane-based compounds or acridine-based compounds conventionally used as a chemiluminescent substrate can be used in the disclosure.

In accordance with a preferred embodiment, the dioxetane-based compounds are dioxetane compounds each with a spiro-adamantane substituent, by way of example, including following compounds but not limited thereto:
AMPPD-(3-(2'-spiroadamatane)-4-methoxy-4-(3"-phosphoryloxy)-phenyl-1,2-di oxetane, CAS=122341-56-4),
CSPD-(3-(2'-(spiro-5-chloroadamantane))-4-methoxy-4-(3"-phosphoryloxy)-phe nyl-1,2-dioxetane, CAS=142456-88-0),
ADP-STAR-(3-(2'-spiroadamatane)-4-methoxy-4-(3"-phosphoryloxy-4"-chloro)-phenyl-1,2-dioxetane, CAS=189942-84-5),
CDP-STAR-(3-(2'-(spiro-5-chloroadamantane))-4-methoxy-4-(3"-phosphoryloxy -4"-chloro)-phenyl-1,2-dioxetane, CAS=160081-62-9), and
TFE-AMPPD-(3-(2'-spiroadamatane)-4-trifluoroethoxy-4-(3"-phosphoryloxy)-p henyl-1,2-dioxetane).

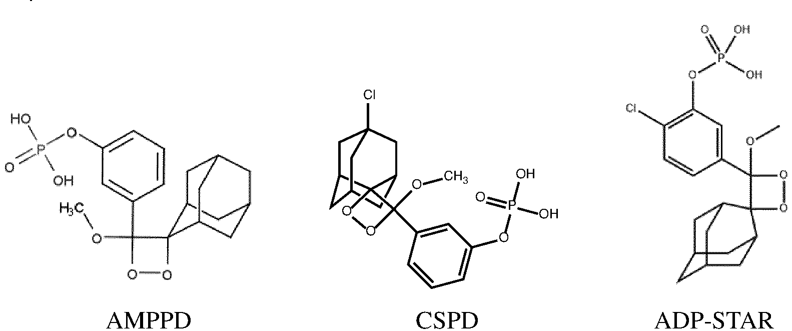

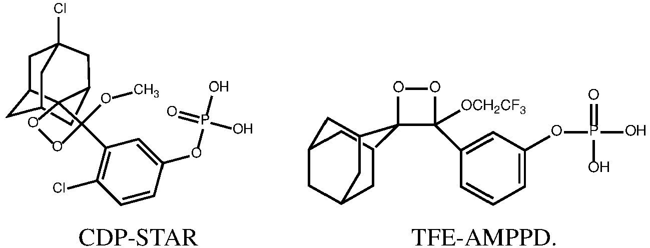

In a specific embodiment, the chemiluminescent substrates present in the form of salts of the above compounds. In accordance with some embodiments, the salts are alkali metal salts, such as sodium salts. Unless otherwise specified, the above compounds mentioned in the text refer to themselves or their salts.

A preferred chemiluminescent substrate is a chlorinated dioxetane-based compound with a spiro-adamantane substituent, such as CSPD, ADP-STAR or CDP-STAR.

A chemiluminescence enhancer containing the above-mentioned carboxyfluorescein is especially suitable for an alkaline phosphatase-catalyzed chemiluminescence system of a dioxetane derivative, and therefore dioxetane compounds are more preferred.

The chemiluminescent substrate in the chemiluminescent substrate solution has a concentration of 50-500 mg/L, preferably 150-250 mg/L, and particularly preferably 200 mg/L.

In accordance with a specific embodiment, the chemiluminescent substrate in the chemiluminescent substrate solution is one of CSPD, ADP-STAR and CDP-STAR, and the surfactant is polyvinylbenzyltrimethylammonium chloride.

In general, the chemiluminescent substrate solution further contains an additive such as a buffer and a preservative.

The type of the buffer is not particularly limited in the disclosure, and all conventionally applicable buffers can be used in the chemiluminescent substrate solution of the disclosure. The buffer may be selected from a Tris buffer solution, an AMP buffer solution (AMP is 2-amino-2-methylpropanol), an AMPD buffer solution (AMPD is 2-amino-2-methyl-1,3-propanediol), a DEA buffer solution (DEA is diethanolamine), a CHES buffer solution (CHES is (2-(N-cyclohexylamino)ethanesulfonic acid), a Mopso buffer solution, an imidazole buffer solution, a phosphate buffer solution, a carbonate buffer solution, a malic acid buffer solution and a glycine buffer solution, but is not limited thereto. By way of example, the buffer is selected from Tris-HCl, AMP-HCl, AMPD-HCl, DEA-HCl, CHES-HCl, boric acid-NaOH, glycine-NaOH. A preferred buffer solution is a Tris-HCl buffer solution.

In accordance with some embodiments, the buffer has a pH of 7.1-10.6, preferably 9-10, and particularly preferably 9.5.

The type of the preservative is not particularly limited in the disclosure, and all preservatives conventionally used in a detection reagent can be used in the disclosure. Examples may be sodium azide, Proclin series, potassium sorbate, sodium benzoate, BND, erythromycin, gentamicin and the like, but are not limited thereto. Sodium azide and Proclin series are preferred.

In accordance with some embodiments, the chemiluminescence enhancer can be packaged individually as a product or can be added to a chemiluminescent substrate solution as an additive component. Therefore, the disclosure further provides a chemiluminescence enhancer including the above-mentioned fluorescent agent and surfactant. Optionally, the chemiluminescence enhancer may further include a necessary additive, such as Tris- HCl and sodium azide.

In accordance with some specific embodiments, the chemiluminescent substrate solution of the disclosure has the compositions below, and is suitable for use as an alkaline phosphatase-catalyzed chemiluminescent system of a dioxetane derivative:

| | |
|---|---|
| a dioxetane derivative | 50-500 mg |
| a carboxyfluorescein | 30-500 mg |
| a water-soluble polymer quaternary ammonium salt | 1-10 g |
| an appropriate amount of a buffer | |
| an appropriate amount of a preservative | |
| water | balanced to 1 L |
| pH value | 9.5 |

In accordance with some embodiments, the chemiluminescence enhancer or the chemiluminescent substrate solution provided by the disclosure is suitable for use in semi-automatic and full-automatic chemiluminescence analyzers used in various chemiluminescence analysis fields, especially in the alkaline phosphatase analysis field. The chemiluminescence enhancer or the chemiluminescent substrate solution of the disclosure has simple formulations, industrially producible materials, controllable costs, and fast luminescence rates.

In accordance with some specific embodiments, the chemiluminescent substrate solution of the disclosure is mixed with a sample in a reaction vessel, an appropriate amount of alkaline phosphatase solution is added by using a full-automatic chemiluminescence immunoassay analyzer and the reaction is incubated for a period of time, and then lighted, the light signals of the substrate liquid in the reaction vessel are collected at different point-in-times, so that the photodynamic curve of the system is obtained.

In accordance with some specific examples, the chemiluminescent substrate solution provided by the disclosure reacts with 5 ng/mL of alkaline phosphatase, and the luminescence value can reach 5,000,000 or more after incubation for 2 min. Moreover, the chemiluminescent substrate can reach plateau in a shorter period of time, and light detection can be performed after 2 min of incubation, whereby the clinical testing throughput can be significantly improved. Further, the background value of the chemiluminescent substrate solution is also reduced to about 1200, which allows the detection of alkaline phosphatase with an ultra-low concentration of 10^-19 mol/L or less, and the detection sensitivity is improved.

The disclosure further provides the use of the above-mentioned carboxyfluorescein in enhancing the chemiluminescence of an alkaline phosphatase-catalyzed dioxetane derivative.

The "sample" mentioned herein, unless otherwise indicated, refers to a biological sample, which may be from a mammal, preferably a blood sample from a human, and more preferably a serum sample.

Various embodiments and advantages of the disclosure are illustrated below by specific examples, but the scope of the disclosure is not limited thereby.

### Example 1: chemiluminescent substrate solutions containing different fluorescent agents

Chemiluminescent substrate solutions were prepared according to the formulation below:
AMPPD: 200 mg
a fluorescent agent: 200 mg
polyvinylbenzyltrimethylammonium chloride: 5 g
Tris-HCl: 50 mM
magnesium chloride: 200 mg
sodium azide: 1 g
water: balanced to 1 L

According to the formulation above, a series of chemiluminescent substrate solutions 1-1 to 1-14 were prepared by using different fluorescent agents, and the species of the fluorescent agent corresponding to each chemiluminescent substrate solution was shown in Table 1.

**Table 1 Chemiluminescent substrate solutions with different fluorescent agents**

| Number | Fluorescent agent |
|---|---|
| 1-1 | 6-carboxyfluorescein |
| 1-2 | Fluorescein isothiocyanate |
| 1-3 | 5-carboxyfluorescein |
| 1-4 | Phloxine B |
| 1-5 | 5(6)carboxvfluorescein |
| 1-6 | 5-aminofluorescein |
| 1-7 | 6-aminofluorescein |
| 1-8 | Solvent red 43 |
| 1-9 | Ervthrosine B |
| 1-10 | Calcein |
| 1-11 | 5(6)nitrofluorescein |
| 1-12 | Pvrenetetrasulfonic acid tetrasodium salt |
| 1-13 | Eosin Y disodium salt |
| 1-14 | Fluorescein sodium |

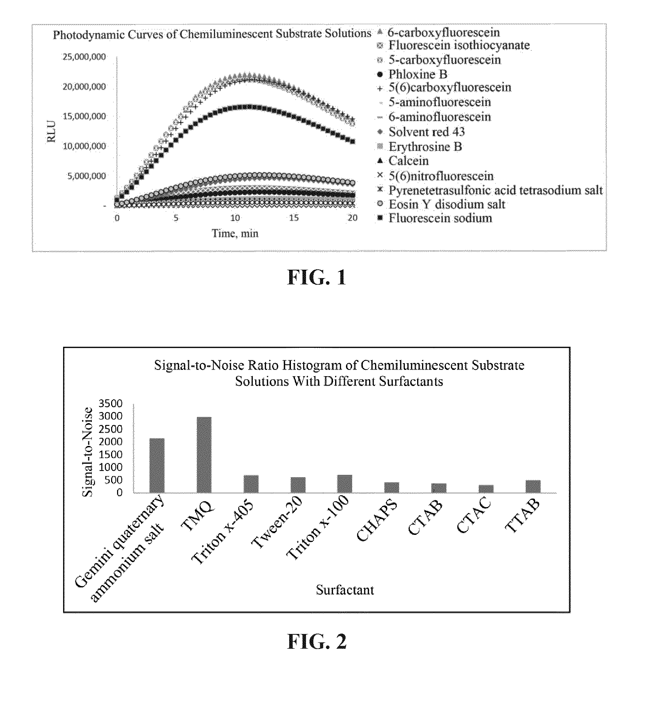

To the above series of chemiluminescent substrate solutions 1-1 to 1-14, tests were performed on Mindray CL-6000i full-automatic chemiluminescence immunoassay analyzer. Each of the chemiluminescent substrate solutions and a certain amount of a solution of magnetic beads marked with 12 ng/mL of alkaline phosphatase were respectively added to a reaction vessel, and after incubation for a period of time, the light signals of the reaction vessel system at different point-of-time were collected and photodynamic curves were plotted (FIG. 1).

From FIG. 1, the luminescence intensity of the systems in which chemiluminescent substrate solutions 1-1, 1-3, and 1-5 were used is much higher than that of the other systems. It is clear that by using a carboxyfluorescein, especially 5-carboxyfluorescein, 6-carboxyfluorescein and 5(6)-carboxyfluorescein as a fluorescent agent of a chemiluminescent substrate solution, the luminescence efficiency of the alkaline phosphatase-catalyzed chemiluminescence system can be greatly improved, and the background signal value was kept relatively low , thereby improving the detection sensitivity.

### Example 2: chemiluminescent substrate solutions containing different surfactants

Chemiluminescent substrate solutions were prepared according to the formulation below:
AMPPD: 200 mg
5(6)-carboxyfluorescein: 200 mg
a surfactant: 5 g
Tris-HCl: 50 mM
magnesium chloride: 200 mg
sodium azide: 1 g
water: balanced to 1 L

According to the formulation above, a series of chemiluminescent substrate solutions 2-1 to 2-9 were prepared by combining different types of surfactants, and the species of the surfactant corresponding to each chemiluminescent substrate solution was shown in Table 2.

**Table 2 Chemiluminescent substrate solutions with different surfactants**

| Number | Surfactant | Signal-to-noise ratio |
|---|---|---|
| 2-1 | Gemini quaternary ammonium salt | 2148 |
| 2-2 | TMQ | 2970 |
| 2-3 | Triton X-405 | 690 |
| 2-4 | Tween-20 | 609 |
| 2-5 | Triton X-100 | 703 |
| 2-6 | CHAPS | 409 |
| 2-7 | CTAB | 365 |
| 2-8 | CTAC | 301 |
| 2-9 | TTAB | 495 |

To the above series of chemiluminescent substrate solutions 2-1 to 2-9, tests were performed on Mindray CL-6000i full-automatic chemiluminescence immunoassay analyzer. Each of the chemiluminescent substrate solutions and a certain amount of a solution of 5 ng/mL alkaline phosphatase were respectively added to a reaction vessel, and after incubation for a period of time, the light signal of the reaction vessel system were collected separately at 6th minute. In another group, only each of the chemiluminescent substrate solutions was added, and the light signal of the reaction vessel system were likewise collected separately at the 6th minute to obtain background signal value. By calculation, signal-to-noise ratios were obtained (signal-to-noise ratio = luminescence signal value / background signal value), as shown in Table 2 (and FIG. 2).

From the data in FIG. 2 and Table 2, the carboxyfluoresceins can combine with different surfactants to prepare chemiluminescent substrate solutions, which are suitable for the alkaline phosphatase-catalyzed chemiluminescence system. he systems with the solutions all have a good luminescence efficiency and a relatively low background signal value. The luminescence intensity of the system in which chemiluminescent substrate solution 2-2 was used is higher than those of the other reagent systems. It is clear that by using TMQ as a surfactant component of a chemiluminescent substrate solution, the luminescence efficiency of an alkaline phosphatase-catalyzed chemiluminescence system can be improved, which is advantageous to the further improvement of detection sensitivity.

### Example 3: chemiluminescent substrate solutions in which a carboxyfluorescein is combined with different chemiluminescent substrates

Chemiluminescent substrate solution 3-1 of CDP-STAR luminescence system was prepared according to the formulation below:
CDP-STAR: 200 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 3-2 of AMPPD luminescence system was prepared according to the formulation below:
AMPPD: 200 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 3-3 of ADP-STAR luminescence system was prepared according to the formulation below:
ADP-STAR: 200 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 3-4 of CSPD luminescence system was prepared according to the formulation below:
CSPD: 200 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

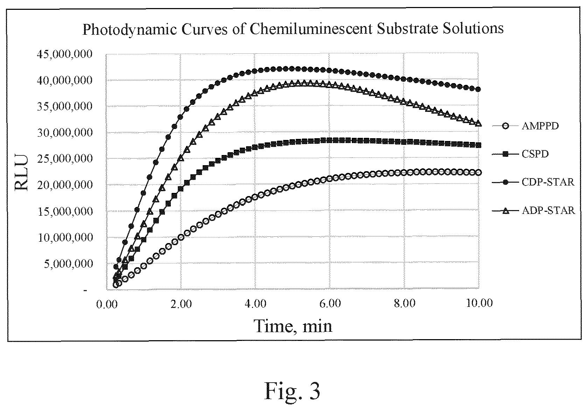

To the above-mentioned chemiluminescent substrate solution reagents 3-1, 3-2, 3-3 and 3-4, tests were performed on Mindray CL-6000i full-automatic chemiluminescence immunoassay analyzer. Each of the chemiluminescent substrate solutions and a certain amount of a solution of 50 ng/mL alkaline phosphatase were respectively added to a reaction vessel, and after incubation for a period of time, the light signals of the reaction vessel system at different point-of-time were collected and photodynamic curves were plotted (FIG. 3).

From FIG. 3, a chemiluminescent substrate solution containing the carboxyfluorescein as a fluorescent agent is suitable for a luminescence system of a dioxetane, such as CDP-STAR luminescence system, AMPPD luminescence system, ADP-STAR luminescence system and CSPD luminescence system. Moreover, the chemiluminescent substrate solutions of the disclosure (i.e., the luminescence system of CDP-STAR or ADP-STAR or CSPD) has a shorter time for reaching plateau and a faster response speed compared with the on-line chemiluminescent substrate solution of AMPPD.

### Example 4: Various formulation designs for chemiluminescent substrate solutions

Chemiluminescent substrate solution 4-1 was prepared according to the formulation below:
CDP-STAR: 200 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 4-2 was prepared according to the formulation below:
CDP-STAR: 50 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 4-3 was prepared according to the formulation below:
CDP-STAR: 500 mg
5(6)carboxyfluorescein: 200 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 4-4 was prepared according to the formulation below:
CDP-STAR: 200 mg
5(6)carboxyfluorescein: 30 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 4-5 was prepared according to the formulation below:
CDP-STAR: 200 mg
5(6)carboxyfluorescein: 500 mg
polyvinylbenzyltrimethylammonium chloride: 5 g
Tris-HCl: 50 mM
magnesium chloride: 200 mg
sodium azide: 1 g
water: balanced to 1 L

Chemiluminescent substrate solution 4-6 was prepared according to the formulation below:
CDP-STAR: 200 mg
5(6)carboxyfluorescein: 200 mg
polyvinylbenzyltrimethylammonium chloride: 1 g
Tris-HCl: 50 mM
magnesium chloride: 200 mg
sodium azide: 1 g
water: balanced to 1 L

Chemiluminescent substrate solution 4-7 was prepared according to the formulation below:
CDP-STAR: 200 mg
5(6)carboxyfluorescein: 200 mg
polyvinylbenzyltrimethylammonium chloride: 10 g
Tris-HCl: 50 mM
magnesium chloride: 200 mg
sodium azide: 1 g
water: balanced to 1 L

To the above-mentioned chemiluminescent substrate solutions 4-1 to 4-7, tests were performed on Mindray CL-6000i full-automatic chemiluminescence immunoassay analyzer. Each of the chemiluminescent substrate solutions and a certain amount of a solution of 5 ng/mL alkaline phosphatase were respectively added to a reaction vessel, and only each of the chemiluminescent substrate solutions was added to a reaction vessel. After incubation for a period of time, the light signals were collected. The background signal values and luminescence signal values at the light collection time of 2 min were obtained. The signal-to-noise ratios were calculated (signal-to-noise ratio = luminescence signal value / background signal value), and the specific data were shown in Table 3 below.

From the data of Table 3, in the alkaline phosphatase-catalyzed chemiluminescence system, all the chemiluminescent substrate solutions 4-1 to 4-7, which contain the carboxyfluorescein of the disclosure and have different formulation designs, have a good luminescence efficiency and a relatively low background signal value, which is advantageous to the improvement of detection sensitivity.

**Table 3 Test results of different chemiluminescent substrate solutions**

| Number | Background signal value | Luminescence signal value | Signal-to-noise ratio |
|---|---|---|---|
| 4-1 | 1249 | 4,030,162 | 3227 |
| 4-2 | 727 | 1,791,183 | 2463 |
| 4-3 | 2810 | 6,045,243 | 2151 |
| 4-4 | 1062 | 2,821,113 | 2657 |
| 4-5 | 1618 | 4,800,302 | 2966 |
| 4-6 | 881 | 1,618,734 | 1836 |
| 4-7 | 1998 | 4,836,194 | 2420 |

## Claims

1. A chemiluminescent substrate solution, comprising a chemiluminescent substrate and a chemiluminescence enhancer, **characterized in that**,
the chemiluminescence enhancer comprises a fluorescent agent and a surfactant, wherein the fluorescent agent comprises one or more carboxyfluoresceins selected from compounds represented by general formula I:

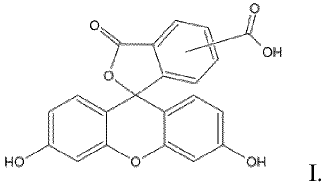

2. The chemiluminescent substrate solution of claim 1, wherein the fluorescent agent is selected from 5-carboxyfluorescein, 6-carboxyfluorescein and 5(6)-carboxyfluorescein; wherein,
5-carboxyfluorescein is 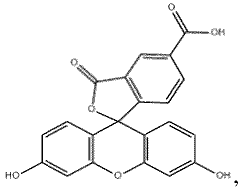
6-carboxyfluorescein is 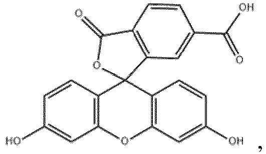
5(6)-carboxyfluorescein is a mixture of 5-carboxyfluorescein and 6-carboxyfluorescein.

3. The chemiluminescent substrate solution of claim 1 or 2, wherein the chemiluminescent substrate is one selected from dioxetane-based compounds and acridine-based compounds, preferably, the chemiluminescent substrate is one selected from dioxetane-based compounds each having a spiro-adamantane substituent, more preferably selected from :
AMPPD: 3-(2'-spiroadamatane)-4-methoxy-4-(3"-phosphoryloxy)-phenyl-1,2-dioxetane, CSPD: 3-(2'-(spiro-5-chloroadamantane))-4-methoxy-4-(3"-phosphoryloxy)-phenyl-1,2-dioxetane,
ADP-STAR: 3-(2'-spiroadamatane)-4-methoxy-4-(3"-phosphoryloxy-4"-chloro)-phenyl-1,2-dioxetane,
CDP-STAR: 3-(2'-(spiro-5-chloroadamantane))-4-methoxy-4-(3"-phosphoryloxy-4"-chloro)-phenyl-1,2-dioxetane, and
TFE-AMPPD: 3-(2'-spiroadamatane)-4-trifluoroethoxy-4-(3"-phosphoryloxy)-phenyl-1,2-dioxetane;
or preferably, the chemiluminescent substrate is one selected from chlorinated dioxetane-based compounds each having a spiro-adamantane substituent, and more preferably selected from CSPD, ADP-STAR and CDP-STAR.

4. The chemiluminescent substrate solution of any one of claims 1 to 3, wherein the surfactant is one or more selected from a cationic surfactant, an anionic surfactant, a zwitterionic surfactant and a non-ionic surfactant, preferably selected from a cationic surfactant of a water-soluble polymer quaternary ammonium salt, a quaternary sulfonium salt and a quaternary phosphonium salt, more preferably selected from the cationic surfactant of the water-soluble polymer quaternary ammonium salt, and more preferably the surfactant is polyvinylbenzyltrimethylammonium chloride.

5. The chemiluminescent substrate solution of any one of claims 1 to 4, wherein,
the chemiluminescent substrate has a concentration of 50-500 mg/L, preferably 150-250 mg/L;
the fluorescent agent has a concentration of 30-500 mg/L, preferably 150-250 mg/L; and
the surfactant has a concentration of 1-10 g/L, preferably 3-7 g/L.

6. The chemiluminescent substrate solution of any one of claims 1 to 5, wherein the chemiluminescent substrate solution further comprises a buffer and a preservative.

7. The chemiluminescent substrate solution of claim 6, wherein the buffer has a pH of 7.1-10.6, and preferably 9-10.

8. The chemiluminescent substrate solution of claim 6 or 7, wherein the buffer is at least one selected from tris(hydroxymethyl)aminomethane-HCl, 2-amino-2-methylpropanol-HCl, 2-amino-2-methyl-1,3-propanediol-HCl, diethanolamine-HCl, 2-(N-cyclohexylamino)ethanesulfonic acid -HCl, boric acid-NaOH and glycine-NaOH.

9. The chemiluminescent substrate solution of any one of claims 6 to 8, wherein the preservative is at least one selected from sodium azide, Proclin series, potassium sorbate and sodium benzoate.

10. Use of the chemiluminescent substrate solution of any one of claims 1 to 9 in an alkaline phosphatase-catalyzed chemiluminescence system.

11. The use of claim 10, wherein the chemiluminescence system contains an alkaline phosphatase having a concentration of less than 10^-19 mol/L.

12. A chemiluminescence enhancer, comprising a fluorescent agent and a surfactant, wherein the fluorescent agent comprises one or more of carboxyfluoresceins selected from compounds represented by general formula I:

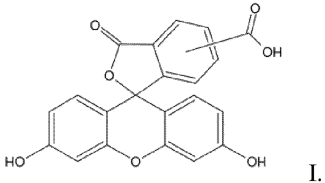

13. The chemiluminescence enhancer of claim 12, wherein the fluorescent agent is selected from 5-carboxyfluorescein, 6-carboxyfluorescein and 5(6)-carboxyfluorescein; wherein,
5-carboxyfluorescein is 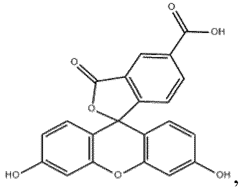
6-carboxyfluorescein is 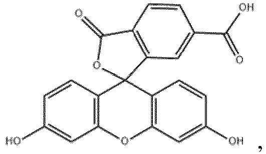 and
5(6)-carboxyfluorescein is a mixture of 5-carboxyfluorescein and 6-carboxyfluorescein.

14. The chemiluminescence enhancer of claim 12 or 13, wherein the surfactant is one or more selected from a cationic surfactant, an anionic surfactant, a zwitterionic surfactant and a non-ionic surfactant, preferably selected from a cationic surfactant of a water-soluble polymer quaternary ammonium salt, a quaternary sulfonium salt or a quaternary phosphonium salt, more preferably selected from the cationic surfactant of the water-soluble polymer quaternary ammonium salt, and more preferably the surfactant is polyvinylbenzyltrimethylammonium chloride.

15. Use of a carboxyfluorescein in enhancing chemiluminescence of a dioxetane derivative catalyzed by alkaline phosphatase, wherein the carboxyfluorescein is selected from compounds represented by general formula I:

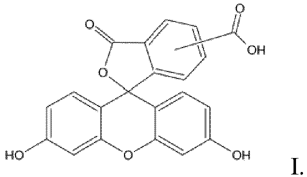

## Patentansprüche

1. Eine chemilumineszierende Substratlösung, die ein chemilumineszierendes Substrat und einen chemilumineszierenden Verstärker umfasst, mit der Eigenschaft, dass der chemilumineszierende Verstärker ein Fluoreszenzmittel und ein Tensid umfasst,
wobei das Fluoreszenzmittel eine oder mehrere Carboxyfluoresceine umfasst, die aus Verbindungen ausgewählt sind, die durch die allgemeine Formel I dargestellt werden:

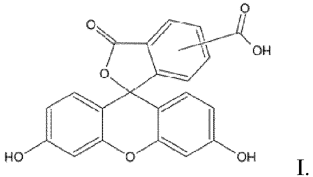

2. Die chemilumineszierende Substratlösung nach Anspruch 1, wobei das Fluoreszenzmittel einer Auswahl aus 5-Carboxyfluorescein, 6-Carboxyfluorescein und 5(6)-Carboxyfluorescein entspricht; wobei:
5-Carboxyfluorescein folgende Formel hat: 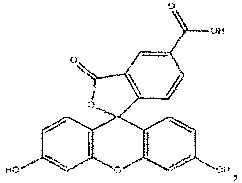
6-Carboxyfluorescein die Formel 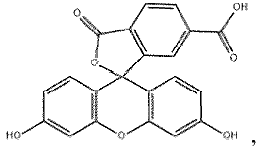
5(6)-Carboxyfluorescein ein Gemisch aus 5-Carboxyfluorescein und 6-Carboxyfluorescein ist.

3. Die chemilumineszierende Substratlösung nach Anspruch 1 oder 2, wobei das chemilumineszierende Substrat ausgewählt ist aus Dioxetan-basierten Verbindungen und Acridin-basierten Verbindungen, vorzugsweise ist das chemilumineszierende Substrat ausgewählt aus Dioxetan-basierten Verbindungen, die jeweils eine Spiro-Adamantan-Substituent enthalten, besonders bevorzugt ausgewählt aus:
AMPPD:
3-(2'-Spiroadamantan)-4-methoxy-4-(3"-phosphoryloxy)-phenyl-1,2-dioxetan,
CSPD:3-(2'-(Spiro-5-chloroadamantan))-4-methoxy-4-(3"-phosphoryloxy)-phenyl-1,2-dioxetan,
ADP-STAR:
3-(2'-Spiroadamantan)-4-methoxy-4-(3"-phosphoryloxy-4"-chloro)-phenyl-1,2-dioxetan,
CDP-STAR:
3-(2'-(Spiro-5-chloroadamantan))-4-methoxy-4-(3"-phosphoryloxy-4"-chloro)-phenyl-1,2-dioxetan, und
TFE-AMPPD:
3-(2'-Spiroadamantan)-4-trifluorethoxy-4-(3"-phosphoryloxy)-phenyl-1,2-dioxetan; oder vorzugsweise ist das chemilumineszierende Substrat ausgewählt aus chlorierten Dioxetan-basierten Verbindungen, die jeweils eine Spiro-Adamantan-Substituent enthalten, und besonders bevorzugt ausgewählt aus CSPD, ADP-STAR und CDP-STAR.

4. Die chemilumineszierende Substratlösung nach einem der Ansprüche 1 - 3, wobei das Tensid ausgewählt ist aus einem oder mehreren kationischen Tensiden, anionischen Tensiden, zwitterionischen Tensiden und nichtionischen Tensiden, vorzugsweise ausgewählt aus einem kationischen Tensid eines wasserlöslichen Polymerquartärammoniumsalzes, einem Quartärsulfoniumsalz und einem Quartärphosphoniumsalz, besodners bevorzugt ausgewählt aus dem kationischen Tensid des wasserlöslichen Polymerquartärammoniumsalzes, und besonders bevorzugt ist das Tensid Polyvinylbenzyltrimethylammoniumchlorid.

5. Die chemilumineszierende Substratlösung nach einem der Ansprüche 1 - 4, wobei: das chemilumineszierende Substrat eine Konzentration von 50-500 mg/L, vorzugsweise 150-250 mg/L hat;
das Fluoreszenzmittel eine Konzentration von 30-500 mg/L, vorzugsweise 150-250 mg/L hat; und
das Tensid eine Konzentration von 1-10 g/L, vorzugsweise 3-7 g/L hat.

6. Die chemilumineszierende - Substratlösung nach einem der Ansprüche 1 - 5, wobei die chemilumineszierende Substratlösung ferner ein Puffer- und ein Konservierungsmittel umfasst.

7. Die chemilumineszierende Substratlösung nach Anspruch 6, wobei der Puffer einen pH-Wert von 7,1 bis 10,6 aufweist, vorzugsweise 9 - 10.

8. Die chemilumineszierende Substratlösung nach Anspruch 6 oder 7, wobei der Puffer mindestens einem der folgenden entspricht: Tris(hydroxymethyl)aminomethan-HCl, 2-Amino-2-methylpropanol-HCl, 2-Amino-2-methyl-1,3-propandiol-HCl, Diethanolamin-HCl, 2-(N-Cyclohexylamino)ethansulfonsäure-HCl, Borsäure-NaOH und Glycin-NaOH.

9. Die chemilumineszierende Substratlösung nach einem der Ansprüche 6 - 8, wobei das Konservierungsmittel mindestens einem der folgenden entspricht: Natriumazid, Proclin-Serie, Kaliumsorbat und Natriumbenzoat.

10. Verwendung der chemilumineszierende Substratlösung nach einem der Ansprüche 1 - 9 in einem durch alkalische Phosphatase katalysierten Chemilumineszenzsystem.

11. Die Verwendung nach Anspruch 10, wobei das Chemilumineszenzsystem eine alkalische Phosphatase mit einer Konzentration von weniger als 10^-19 mol/L enthält.

12. Ein chemilumineszierender Verstärker mit einem Fluoreszenzmittel und einem Tensid, wobei das Fluoreszenzmittel eine oder mehrere Carboxyfluoresceine umfasst, ausgewählt aus Verbindungen, die durch die allgemeine Formel I dargestellt sind:

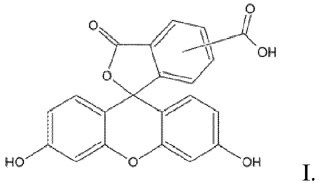

13. Der chemilumineszierender Verstärker nach Anspruch 12, wobei das Fluoreszenzmittel ausgewählt ist aus 5-Carboxyfluorescein, 6-Carboxyfluorescein und 5(6)-Carboxyfluorescein; wobei:
5-Carboxyfluorescein folgende Formel hat: 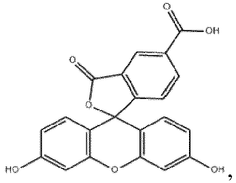
6-Carboxyfluorescein die Formel 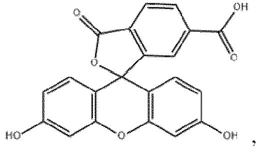
5(6)-Carboxyfluorescein ein Gemisch Aus 5-Carboxyfluorescein und 6-Carboxyfluorescein ist.

14. Der chemilumineszierender Verstärker nach Anspruch 12 oder 13, wobei das Tensid ausgewählt aus einem oder mehreren kationischen Tensiden, anionischen Tensiden, zwitterionischen Tensiden und nichtionischen Tensiden ist, vorzugsweise ausgewählt aus einem kationischen Tensid eines wasserlöslichen Polymerquartärammoniumsalzes, einem Quartärsulfoniumsalz oder einem Quartärphosphoniumsalz, besonders bevorzugt ausgewählt aus dem kationischen Tensid des wasserlöslichen Polymerquartärammoniumsalzes, und besonders bevorzugt das Tensid Polyvinylbenzyltrimethylammoniumchlorid ist.

15. Verwendung eines Carboxyfluoresceins zur Verstärkung der Chemilumineszenz eines durch alkalische Phosphatase katalysierten Dioxetan-Derivats, wobei das Carboxyfluorescein ausgewählt ist aus Verbindungen, die durch die allgemeine Formel I dargestellt sind:

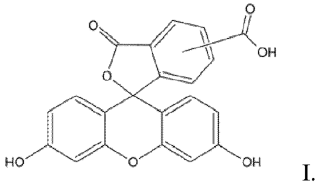

## Revendications

1. Solution de substrat chimiluminescent, comprenant un substrat chimiluminescent et un activateur de chimioluminescence, **caractérisée en ce que**,
l'activateur de chimioluminescence comprend un agent fluorescent et un tensioactif, où l'agent fluorescent comprend une ou plusieurs carboxyfluorescéines sélectionnées parmi les composés représentés par la formule générale I :

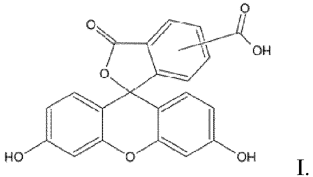

2. Solution de substrat chimiluminescent selon la revendication 1, dans laquelle l'agent fluorescent est sélectionné parmi la 5-carboxyfluorescéine, la 6-carboxyfluorescéine et la 5(6)-carboxyfluorescéine ; où,
la 5-carboxyfluorescéine est 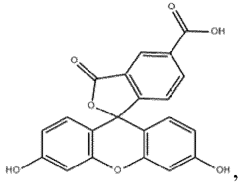
la 6-carboxyfluorescéine est 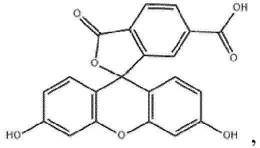
la 5(6)-carboxyfluorescéine est un mélange de la 5-carboxyfluorescéine et de la 6-carboxyfluorescéine.

3. Solution de substrat chimiluminescent selon la revendication 1 ou 2, dans laquelle le substrat chimiluminescent est un composé sélectionné parmi des composés à base de dioxétane et des composés à base d'acridine, de préférence, le substrat chimiluminescent est un composé sélectionné parmi des composés à base de dioxétane ayant chacun un substituant de spiro-adamantane, de façon davantage préférée sélectionné parmi :
AMPPD :
3-(2'-spiroadamantane)-4-méthoxy-4-(3"-phosphoryloxy)-phényl-1,2-dioxétane,
CSPD : 3-(2'-(spiro-5-adamantane))-4-méthoxy-4-(3"-phosphoryloxy)-phényl-1,2-dioxétane,
ADP-STAR :
3-(2'-spiroadamantane)-4-méthoxy-4-(3"-phosphoryloxy-4"-chloro)-phényl-1,2-dioxétane,
CDP-STAR :
3-(2'-(spiro-5-chloroadamantane))-4-méthoxy-4-(3"-phosphoryloxy-4"-chloro)-phényl-1,2-dioxétane, et
TFE-AMPPD :
3-(2'-spiroadamantane)-4-trifluoroéthoxy-4-(3"-phosphoryloxy)-phényl-1,2-dioxétane ;
ou de préférence, le substrat chimiluminescent est un composé sélectionné parmi des composés à base de dioxétane chlorés présentant chacun un substituant de spiro-adamantane, et de façon davantage préférée sélectionné parmi le CSPD, l'ADP-STAR et le CDP-STAR.

4. Solution de substrat chimiluminescent selon l'une quelconque des revendications 1 à 3, dans laquelle le tensioactif est un ou plusieurs tensioactifs sélectionnés parmi un tensioactif cationique, un tensioactif anionique, un tensioactif zwitterionique et un tensioactif non ionique, de préférence sélectionné parmi un tensioactif cationique d'un sel d'ammonium quaternaire polymère soluble dans l'eau, d'un sel de sulfonium quaternaire et d'un sel de phosphonium quaternaire, de façon davantage préférée sélectionné parmi le tensioactif cationique du sel d'ammonium quaternaire polymère soluble dans l'eau, et de façon davantage préférée le tensioactif est le polychlorure de vinylbenzyltriméthylammonium.

5. Solution de substrat chimiluminescent selon l'une quelconque des revendications 1 à 4, dans laquelle,
le substrat chimiluminescent présente une concentration de 50 à 500 mg/L, de préférence de 150 à 250 mg/L ;
l'agent fluorescent présente une concentration de 30 à 500 mg/L, de préférence de 150 à 250 mg/L ; et
le tensioactif présente une concentration de 1 à 10 g/L, de préférence de 3 à 7 g/L.

6. Solution de substrat chimiluminescent selon l'une quelconque des revendications 1 à 5, où la solution de substrat chimiluminescent comprend en outre un tampon et un agent de conservation.

7. Solution de substrat chimiluminescent selon la revendication 6, dans laquelle le tampon présente un pH de 7,1 à 10,6, et de préférence de 9 à 10.

8. Solution de substrat chimiluminescent selon la revendication 6 ou 7, dans laquelle le tampon est au moins un composé sélectionné parmi le tris(hydroxyméthyl)aminométhane-HCl, le 2-amino-2-méthylpropanol-HCl, le 2-amino-2-méthyl-1,3-propanediol-HCl, le diéthanolamine-HCl, l'acide 2-(N-cyclohexylamino)éthanesulfonique-HCl, l'acide borique-NaOH et le glycine-NaOH.

9. Solution de substrat chimiluminescent selon l'une quelconque des revendications 6 à 8, dans laquelle l'agent de conservation est au moins un composé sélectionné parmi l'azoture de sodium, la série Proclin, le sorbate de potassium et le benzoate de sodium.

10. Utilisation d'une solution de substrat chimiluminescent selon l'une quelconque des revendications 1 à 9 dans un système de chimioluminescence catalysée par phosphatase alcaline.

11. Utilisation selon la revendication 10, où le système de chimioluminescence contient une phosphatase alcaline présentant une concentration de moins de 10⁻¹⁹ mol/L

12. Activateur de chimioluminescence, comprenant un agent fluorescent et un tensioactif, où l'agent fluorescent comprend une ou plusieurs carboxyfluorescéines sélectionnées parmi les composés représentés par la formule générale I :

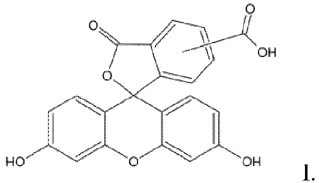

13. Activateur de chimioluminescence selon la revendication 12, dans lequel l'agent fluorescent est sélectionné parmi la 5-carboxyfluorescéine, la 6-carboxyfluorescéine et la 5(6)-carboxyfluorescéine ; où,
la 5-carboxyfluorescéine est 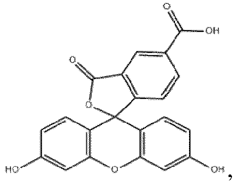
la 6-carboxyfluorescéine est 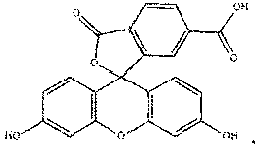
la 5(6)-carboxyfluorescéine est un mélange de la 5-carboxyfluorescéine et de la 6-carboxyfluorescéine.

14. Activateur de chimioluminescence selon la revendication 12 ou 13, dans lequel le tensioactif est un ou plusieurs tensioactifs sélectionnés parmi un tensioactif cationique, un tensioactif anionique, un tensioactif zwitterionique et un tensioactif non ionique, de préférence sélectionné parmi un tensioactif cationique d'un sel d'ammonium quaternaire polymère soluble dans l'eau, d'un sel de sulfonium quaternaire ou d'un sel de phosphonium quaternaire, de façon davantage préférée sélectionné parmi le tensioactif cationique du sel d'ammonium quaternaire polymère soluble dans l'eau, et de façon davantage préférée le tensioactif est le polychlorure de vinylbenzyltriméthylammonium.

15. Utilisation d'une carboxyfluorescéine dans l'activation de chimioluminescence d'un dérivé de dioxétane catalysée par phosphatase alcaline, où la carboxyfluorescéine est sélectionnée parmi les composés représentés par la formule générale I :
